# EUROPEAN PATENT APPLICATION

(11) **EP 2 444 111 A1**
(43) Date of publication of application: **25.04.2012**
(21) Application number: 11185736.3
(22) Date of filing: 19.10.2011
(51) Int. Cl.: A61M 5/32

(54) **Multipurpose protective cap for syringes**

(30) Priority: 25.10.2010 IT MI20100322 U
(71) Applicant: Palli, Daniela, 20149 Milano (IT)
(72) Inventor: Palli, Daniela, 20149 Milano (IT)
(74) Representative: Cicogna, Franco

(57) **Abstract**

A multipurpose protective cap construction for syringes, characterized in that said syringe needle cap comprises a tube body designed for covering an overall length of a syringe needle or other needle instrument, said tube body having a tube body mouth portion including a funnel device adapted to operate as a lead-in member for engaging the needle in the tube body and a protective screen member for protecting the fingers of a hand gripping said tube body.

## Description

### BACKGROUND OF THE INVENTION

The present invention relates to a multipurpose protective cap for syringes.

As known, medical syringes conventionally comprise a protective cap for protecting the syringe needle.

The cap also operates for covering the syringe needle after use, for preventing the needle, as the syringe is disposed of, from injuring the disposal operators.

In actual practice, the needle cap is arranged on the syringe needle immediately after having used the latter, by merely threading the syringe needle through the protective cap mouth.

Such an operation is performed by gripping the cap by one hand and the syringe by the other.

However, the cap mouth is a comparatively narrow opening and since the needle is covered with a comparatively high covering rate, it frequently occurs that the mouth portion is not precisely "centered", thereby the operator's hand may be injured.

This would be very dangerous because of possible infections the syringe needle may transmit to the operator.

In this connection it should be pointed out that operators performing a large number of injections, for example in a hospital, may be frequently injured by the syringe needle as the latter is covered by the needle cap, thereby being easily and undesirably infected by said needle.

Thus, it would be desirable to overcome the above mentioned drawback, or at least to greatly reduce possible prickings to the operator as the syringe needle cap is rearranged on said needle.

### SUMMARY OF THE INVENTION

Accordingly, the aim of the present invention is to provide a syringe needle covering cap construction, which is adapted to eliminate or at least to greatly reduce any dangers of pricking the hands of the syringe operator.

Within the scope of the above mentioned aim, a main object of the invention is to provide such a multipurpose syringe cap construction which can be applied to any desired type of syringes or other needle instruments having needles to be introduced into a patient vessel, and conventionally called "butterfly needles".

Another object of the present invention is to provide such a multipurpose syringe needle cap construction which may be easily made starting from easily available elements and materials and which, moreover, is very competitive from a mere economic standpoint.

Yet another object of the present invention is to provide such a multipurpose syringe needle cap which is very reliable and safe in operation.

According to one aspect of the present invention, the above mentioned aim and objects, as well as further objects, which will become more apparent hereinafter, are achieved by a multipurpose protective syringe needle cap, characterized in that said syringe needle cap comprises a tube body designed for covering an overall length of a syringe needle or other needle instrument, said tube body having a tube body mouth portion including a funnel member adapted to operate as a lead-in member for engaging the needle in the tube body and a protective screen member for protecting the fingers of a hand gripping said tube body.

Said funnel device has a substantially truncated cone, half-spherical or other suitable tapering configuration.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:
Figure 1 is a partially exploded perspective view showing a protective cap construction according to the present invention and a related syringe;
Figure 2 is a further perspective view, similar to Figure 1, showing the cap construction engaged on the needle;
Figure 3 is a further partially exploded perspective view showing the protective cap construction with a lead-in member or device of different configuration, according to the present invention, and a related syringe;
Figure 4 is a further perspective view, similar to Figure 3, showing the protective cap construction in a needle engagement condition thereof;
Figure 5 is a detailed enlarged view showing a detail of the protective cap construction of Figures 1 and 2, in a needle engagement condition thereof; and
Figure 6 is a further enlarged view showing in a more detailed manner the protective cap construction of Figures 3 and 4, in a needle engagement condition thereof.

### DESCRIPTION OF THE PREFERRED EMBODIMENTS

With reference to the number references of the above mentioned figures, the multipurpose protective cap construction according to the present invention, which has been generally indicated by the reference number 1, comprises a tube or tubular body 2 designed for covering the overall length of a needle 3 of a syringe 4.

According to the present invention, the tube or tubular body 2 comprises a lead-in portion or mouth having a funnel device or member 10.

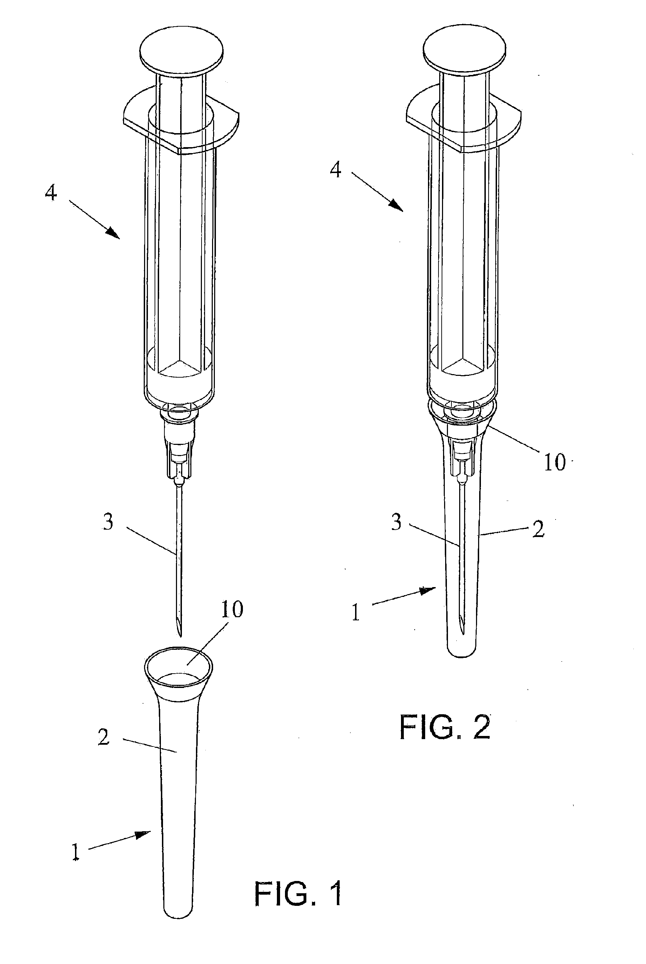

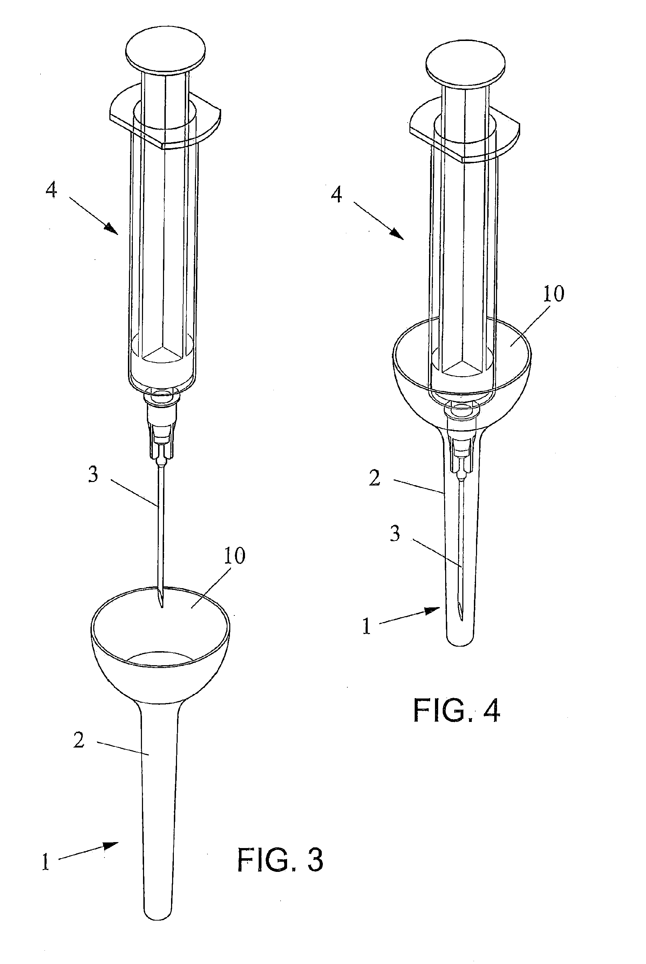

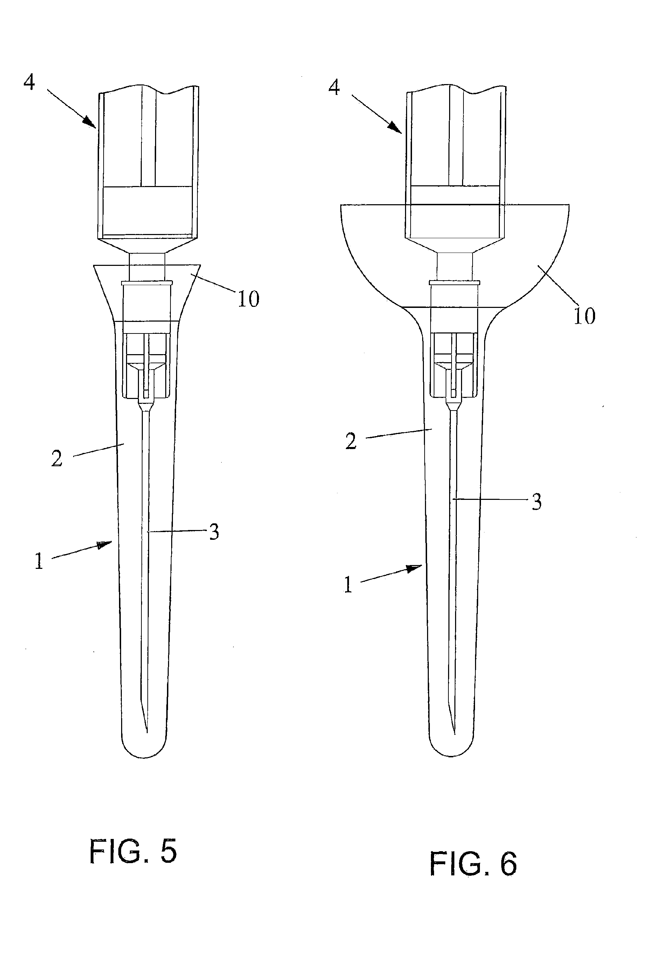

As clearly shown in Figures 1, 2 and 5, the funnel member 10 has a substantially truncated cone configuration or, as shown in Figures 3, 4 and 6, a half-spherical or ball configuration, or any other suitable tapering shape.

In each case, said funnel device or member 10 is adapted to operate as a lead-in member for engaging the tip of the needle 3 therein, and is moreover provided with a protective screen member 5 for protecting the fingers of a hand gripping the protective cap construction for removing the latter from the needle.

The protective member or device according to the present invention may also be applied to needles designed for puncturing a vein of a patient, in particular the so-called butterfly needles.

From the above disclosure it should be apparent that the invention fully achieves the intended aim and objects: in fact, the invention has provided a protective cap construction having a funnel protective device or member adapted to protect the fingers of an operator's hand in disposing of the needle after use.

In particular the above disclosed funnel device or member allows the needle to be easily engaged in its protective cap, while preventing said needle from pricking the operator's fingers.

In this connection, it should be pointed out that the funnel device or member 10 may be made in a single piece with the tube or tubular body 2, for example by injection molding any suitable plastics materials.

It has been found that the invention fully achieves the intended aim and objects.

In practicing the invention, the used materials, as well as the contingent size and shapes, can be any, depending on requirements.

## Claims

1. A multipurpose protective cap construction for syringes, **characterized in that** said syringe needle cap comprises a tube body designed for covering an overall length of a syringe needle or other needle instrument, said tube body having a tube body mouth portion including a funnel device adapted to operate as a lead-in member for engaging the needle in the tube body and a protective screen member for protecting the fingers of a hand gripping said tube body.

2. A multipurpose protective cap construction for syringes, according to claim 1, **characterized in that** said funnel device has a substantially truncated cone configuration.

3. A multipurpose protective cap construction for syringes, according to claim 1, **characterized in that** said funnel device has a half-spherical configuration.

4. A multipurpose protective cap construction for syringes, according to claim 1, **characterized in that** said funnel device has a tapering configuration.

5. A multipurpose protective cap construction for syringes, according to claim 1, **characterized in that** said funnel device has a protective screen and said screen is made as a single piece with the tube body.
